# EUROPÄISCHE PATENTSCHRIFT

(11) **EP 1 773 581 B1**
(45) Veröffentlichungstag und Bekanntmachung des Hinweises auf die Patenterteilung: **21.03.2012**
(21) Anmeldenummer: 05766088.8
(22) Anmeldetag: 20.07.2005
(51) Int. Cl.: B32B 5/14, B32B 25/14, A41D 31/00, A61F 13/66

(54) **ELASTISCHES LAMINAT**
ELASTIC LAMINATE
STRATIFIE ELASTIQUE

(30) Priorität: 21.07.2004 DE 102004035396
(43) Veröffentlichungstag der Anmeldung: 18.04.2007
(73) Patentinhaber: Nordenia Deutschland Gronau GmbH, 48599 Gronau (DE)
(72) Erfinder: HAGEMANN, Andreas, 46414 Rhede (DE); NIEPELT, Ralf, 48599 Gronau (DE); SOLLMANN, Henner, 48599 Gronau (DE); BALDAUF, Georg, 48366 Laer (DE)
(74) Vertreter: Albrecht, Rainer Harald
(86) Internationale Anmeldenummer: PCT/EP2005/007917
(87) Internationale Veröffentlichungsnummer: WO 2006/008149

(56) Entgegenhaltungen:
- EP-A- 1 437 112
- WO-A-01/32403
- WO-A-01/47710
- WO-A-94/00292
- WO-A-95/19258
- WO-A-96/34741

## Beschreibung

Die Erfindung betrifft ein elastisches Laminat, bestehend aus
a) einer coextrudierten elastischen Folie, die eine elastomere Trägerschicht und mindestens eine Deckschicht aufweist und
b) mindestens einer textilen Außenschicht aus Nonwoven,
wobei die Deckschicht eine im Vergleich zur Trägerschicht geringe Schichtdicke aufweist und die Außenschicht auf die Deckschicht aufkaschiert ist. Ein solches elastisches Laminat eignet sich für Verschlussbänder und elastische Bündchen an Babywindeln. Aus dem elastischen Laminat kann ferner elastisches Verbandmaterial gefertigt werden. Eine weitere Anwendungsmöglichkeit ist die Verwendung als elastischer Streifen in Kappen, Sporthüllen und dergleichen.

Ein elastisches Laminat mit den beschriebenen Merkmalen ist aus DE 298 25 018 U1 bekannt. Das Laminat enthält eine coextrudierte elastische Folie, die eine elastomere Trägerschicht und nicht klebrige Deckschichten aufweist. Die Deckschichten bestehen aus halbkristallinen oder amorphen Polymeren, die sich weniger elastomer verhalten als die Trägerschicht. Vorzugsweise sind die Deckschichten nicht elastisch und bestehen aus Polyolefinen. Die im Wesentlichen plastisch verformbaren Deckschichten sollen eine gute Haftung zwischen dem elastomeren Material der Trägerschicht und der textilen Außenschicht herstellen und werden im Rahmen der herrschenden Lehre auch für erforderlich gehalten, damit die elastische Folie von einer Rolle abgezogen, einem Kaschierwerk zugeführt und dort mit einer Nonwovenschicht verbunden werden kann. Die nicht elastomeren Deckschichten wirken sich jedoch nachteilig auf das Dehnungsverhalten des elastischen Laminats aus. Sie tragen dazu bei, dass sich das elastische Laminat nach einer Dehnung nicht vollständig zurückstellt und eine bleibende Dehnung aufweist. Der Effekt ist umso größer je dicker und steifer die Deckschichten sind. Im Rahmen der bekannten Maßnahmen versucht man daher, möglichst dünne Deckschichten aufzubringen und den negativen Effekt der plastisch dehnbaren Deckschichten durch eine dickere elastomere Trägerschicht zu kompensieren.

Vor diesem Hintergrund liegt der Erfindung die Aufgabe zugrunde, die Elastizität des Laminats zu verbessern. Die das Dehnungsverhalten des Laminats bestimmende elastische Folie soll eine geringe Foliendicke aufweisen und sich nach einer Dehnung möglichst vollständig wieder zurückstellen.

Die Aufgabe wird erfindungsgemäß dadurch gelöst, dass die Deckschicht der elastischen Folie aus einem elastomeren Material besteht, welches ebenso wie die elastomere Trägerschicht nach Zugbeanspruchung bei Raumtemperatur im Gegensatz zu Thermoplasten einen ausgeprägten elastischen Anteil besitzt und Zusätze zur Modifizierung der Oberflächeneigenschaften enthält. Mit dem bloßen Auge sind an der Oberfläche der Folie nach einer reversiblen Dehnung um z. B. 400 % keine Aufwerfungen, Mikrotexturen oder sonstige Strukturen, die auf eine Veränderung der Folie hindeuten, feststellbar. Durch geeignete Zusätze in den Deckschichten kann die Oberfläche der Folie so modifiziert werden, dass die Folie nicht klebrig ist und gut verarbeitet werden kann. Durch den mehrschichtigen Aufbau der Folie werden die Zusätze wirkungsvoll in den Randzonen der elastischen Folie konzentriert. Die bezogen auf die Gesamtmasse der elastischen Folie kleinen Mengen an Zusätzen zur Modifizierung der Oberflächeneigenschaften wirken sich auf die Dehnungseigenschaften der elastischen Folie nicht aus.

Das elastomere Material für die Deckschicht und die Trägerschicht kann aus einem thermoplastischen Elastomer aus der Gruppe der Block-Copolymere, der elastomeren Polyurethane oder auch aus Mischungen dieser Elastomere untereinander bestehen. Als Block-Copolymere eignen sich insbesondere Styrol/Isopren-,Butadien- oder Etyhlen-Butylen/Styrol-Block-Copolymere (SIS, SBS oder SEBS). Als olefinische Elastomere sind beispielsweise Ethylen-Propylen-Elastomere, Ethylen-Propylen-Dien-Polymer-Elastomere und Metallocen-Polyolefin-Elastomere einsetzbar. Elastomere Zusammensetzungen können ferner auch Ethylen-Vinylacetat-Elastomere enthalten. Das elastomere Material der Deckschicht kann dem elastomeren Material der Trägerschicht entsprechen.

Die Trägerschicht kann im Querschnitt eine homogene Zusammensetzung oder auch Zonen unterschiedlicher Zusammensetzung aufweisen. Im letzteren Fall kann durch die stoffliche Zusammensetzung und Dicke der Zonen das Elastizitätsverhalten der Trägerschicht modifiziert werden. Eine im Querschnitt aus mehreren Zonen bestehende Trägerschicht lässt sich auf einfache Weise beispielsweise dadurch herstellen, dass eine mehrschichter, extrudierter Folienschlauch bei hohen Temperaturen zu einer flachen Folienbahn zusammengelegt wird, wobei die innenliegenden Schichten des Folienschlauches zu einer Kernzone der flachgelegten Folienbahn verschmelzen.

Ferner kann die Trägerschicht Zusätze zur Verbesserung der Klebefähigkeit zwischen Trägerschicht und Deckschicht enthalten. Sofern die Trägerschicht Zonen aufweist, die sich hinsichtlich ihrer stofflichen Zusammensetzung unterscheiden, sind die Zusätze vorzugsweise nur in den an die Deckschicht angrenzenden Randzonen der Trägerschicht enthalten. Die Deckschicht enthält vorzugsweise Antiblockmittel und/oder Gleitmittel als Zusätze zur Modifizierung der Oberflächeneigenschaften. Die Zusätze sollen eine entblockende Wirkung haben und die Verklebung mit dem Nonwoven nicht wesentlich erschweren. Als Zusätze eignen sich polymere Zusätze, insbesondere Polyolefine, Copolymere der Polyolefine und das Polystyrol. Generell sind Polymere geeignet, die mit dem Elastomer mehr oder weniger unverträglich sind. Als Zusätze können ferner anorganische Füllstoffe, insbesondere Talkum oder Kreide eingesetzt werden. Ferner sind Antiblockmittel, z. B. Siliciumdioxide, Silikate, Calciumcarbonate geeignet. Als Gleitmittel können schließlich auch Fettsäureamide einge-, setzt werden. Die genannten Zusätze können als alleinige Komponente oder in Mischungen verwendet werden. Gemäß einer bevorzugten Ausführung der Erfindung bestehen die Zusätze aus Polystyrol oder einer Mischung aus Talkum und Polypropylen oder der Kombination aus EVA und einem Gleitmittel. Der mengenmäßige Anteil der Zusätze in der Deckschicht ist anhand weniger orientierender Versuche festlegbar. Der Anteil der in der Deckschicht enthaltenden Zusätze ist kleiner als 50 Gew.-%, bezogen auf die Masse der Deckschicht. Vorzugsweise ist der Anteil der in der Deckschicht enthaltenden, nicht elastomeren Zusätze kleiner als 30 Gew.-%, bezogen auf die Masse der Deckschicht.

Die elastomere Trägerschicht kann eine Dicke zwischen 20 µm und 200 µm aufweisen. Die Dicke der elastomeren Deckschicht ist vorzugsweise kleiner als 20 µm.

Die Verbindung zwischen der textilen Außenschicht und der Deckschicht kann auf verschiedene Weise erfolgen. Eine erste bevorzugte Ausführung sieht vor, dass die textile Außenschicht mit der Deckschicht der elastischen Folie flächig verklebt ist. Ferner liegt es im Rahmen der Erfindung, dass die textile Außenschicht an punktförmigen oder linienförmigen Kontaktflächen mit der textilen Außenschicht verklebt oder durch Thermobondieren verbunden ist.

"Nonwoven" bezeichnet eine Vliesstofflage aus richtungsorientierten oder zufällig angeordneten Fasern, die durch Reibung und/oder Kohäsion und/oder Adhäsion verbunden sind. Die Vliesstofflage kann mechanisch verdichtet sein. Ferner können die Fasern thermisch oder durch einen pulverförmig eingebrachten Schmelzklebstoff gebunden sein. Das Nonwoven-Material der Außenschicht kann aus elastomeren Fasern bestehen. Eine andere Ausführung der Erfindung sieht vor, dass das Nonwoven-Material der Außenschicht aus nicht elastomeren Fasern besteht, die nach einer Verstreckung eine signifikante bleibende Verformung aufweisen, und dass die Außenschicht einen dehnbaren Bereich aufweist, der durch Querverstreckung des Laminats in einem mäanderförmigen Walzenspalt zwischen zwei ineinandergreifenden Profilwalzen erzeugt worden ist. Der dehnbare Bereich der Außenschicht ist zweckmäßig so bemessen, dass das Laminat mit einer Dehnungskraft von weniger als 4 N bezogen auf einen Probenstreifen von einem cm Breite um mindestens 100 % dehnbar ist.

Gemäß einer bevorzugten Ausführungsform der Erfindung weist die elastische Folie eine Trägerschicht sowie beidseits der Trägerschicht eine elastische Deckschicht auf und ist auf beiden Seiten der elastischen Folie jeweils eine textile Außenschicht aus Nonwoven aufkaschiert. Die Trägerschicht der elastischen Folie kann im Querschnitt eine homogene Zusammensetzung oder auch mehrere Zonen, die sich hinsichtlich der stofflichen Zusammensetzung unterscheiden, aufweisen.

## Patentansprüche

1. Elastisches Laminat für Verschlussbänder oder elastische Bündchen an Windeln bestehend aus
einer coextrudierten elastischen Folie, die eine elastomere Trägerschicht und mindestens eine Deckschicht aufweist, und
mindestens einer textilen Außenschicht aus Nonwoven,
wobei die Deckschicht eine im Vergleich zur Trägerschicht geringe Schichtdicke aufweist, wobei die Außenschicht auf die Deckschicht aufkaschiert ist und wobei die Deckschicht aus einem elastomeren Material besteht, welches ebenso wie die elastomere Trägerschicht im Gegensatz zu Thermoplasten nach Zugbeanspruchung bei Raumtemperatur einen ausgeprägten elastischen Anteil besitzt und Zusätze zur Modifizierung der Oberflächeneigenschaften enthält, **dadurch gekennzeichnet, dass** das elastomere Material für die Deckschicht und die Trägerschicht aus einem thermoplastischen Elastomer aus der Gruppe der Block-Copolymere, der elastomeren Polyurethane oder aus Mischungen dieser Elastomere besteht, dass das Nonwoven-Material der Außenschicht aus nicht elastomeren Fasern besteht, die nach einer Verstreckung eine signifikante bleibende Verformung aufweisen, und dass die Außenschicht einen dehnbaren Bereich aufweist, der durch Querverstreckung des Laminates in einem mäanderförmigen Walzenspalt zwischen zwei ineinander greifenden Profilwalzen erzeugt worden ist.

2. Elastisches Laminat nach Anspruch 1, **dadurch gekennzeichnet, dass** das elastomere Material der Deckschicht dem elastomeren Material der Trägerschicht entspricht.

3. Elastisches Laminat nach einem Anspruch 1 oder 2, **dadurch gekennzeichnet, dass** die Trägerschicht Zusätze zur Verbesserung der Klebefähigkeit zwischen Trägerschicht und Deckschicht enthält.

4. Elastisches Laminat nach einem der Ansprüche 1 bis 3, **dadurch gekennzeichnet, dass** die Deckschicht Antiblockmittel und/oder Gleitmittel als Zusätze zur Modifizierung der Oberflächeneigenschaften enthält.

5. Elastisches Laminat nach einem der Ansprüche 1 bis 4, **dadurch gekennzeichnet, dass** die Deckschicht als Zusätze Polymere, vorzugsweise Polyolefine, Copolymere der Polyolefine oder Polystyrol, anorganische Füllstoffe, anorganische Antiblockiermittel, Gleitmittel oder Mischungen aus diesen Stoffen enthält.

6. Elastisches Laminat nach Anspruch 4 oder 5, **dadurch gekennzeichnet, dass** der Anteil der in der Deckschicht enthaltenen nicht elastomeren Zusätze kleiner ist als 50 Gew.-%, vorzugsweise kleiner als 30 Gew.-%, jeweils bezogen auf die Masse der Deckschicht.

7. Elastisches Laminat nach einem der Ansprüche 1 bis 6, **dadurch gekennzeichnet, dass** die elastomere Trägerschicht eine Dicke zwischen 20 µm und 200 µm aufweist und dass die Dicke der elastomeren Deckschicht kleiner als 20 µm ist.

8. Elastisches Laminat nach einem der Ansprüche 1 bis 7, **dadurch gekennzeichnet, dass** die textile Außenschicht flächig mit der Deckschicht der elastischen Folie verklebt ist.

9. Elastisches Laminat nach einem der Ansprüche 1 bis 8, **dadurch gekennzeichnet, dass** die textile Außenschicht an punktförmigen oder linienförmigen Kontaktflächen mit der textilen Außenschicht verklebt oder durch Thermobondieren verbunden ist.

10. Elastisches Laminat nach einem der Ansprüche 1 bis 9, **dadurch gekennzeichnet, dass** der dehnbare Bereich der Außenschicht so bemessen ist, dass das Laminat mit einer Dehnungskraft von weniger als 4 N/cm um mindestens 100 % dehnbar ist.

11. Elastisches Laminat nach einem der Ansprüche 1 bis 10, **dadurch gekennzeichnet, dass** die elastische Folie dreischichtig ausgebildet ist und auf beiden Seiten der elastischen Folie jeweils eine textile Außenschicht aus Nonwoven aufkaschiert ist.

## Claims

1. Elastic laminate for closure tapes or elastic edgings on nappies consisting of
a co-extruded elastic film that has an elastomer support layer and at least one cover layer, and
at least one textile outer layer made of a nonwoven,
wherein the cover layer has a low layer thickness compared to the support layer, wherein the outer layer is laminated onto the cover layer and wherein the cover layer consists of an elastomer material, which, like the elastomer support layer, possesses a marked elastic component after tensile stress at room temperature, in contrast to thermoplastics, and contains additives for modifying the surface properties **characterised in that** the elastomer material for the cover layer and the support layer consists of a thermoplastic elastomer from the group of block copolymers, elastomer polyurethanes or of mixtures of these elastomers, that the nonwoven material of the outer layer consists of non-elastomeric fibres which have a significant permanent deformation following a stretching and that the outer layer has a stretchable region which has been produced by transverse stretching of the laminate in a meander-shaped roller nip between intermeshing profile rollers.

2. Elastic laminate according to claim 1, **characterised in that** the elastomer material of the cover layer corresponds to the elastomer material of the support layer.

3. Elastic laminate according to claim 1 or 2, **characterised in that** the support layer contains additives for improving the adhesion capacity between support layer and cover layer.

4. Elastic laminate according to any one of claims 1 to 3, **characterised in that** the cover layer contains anti-blocking agents and/or lubricants as additives for modification of the surface properties.

5. Elastic laminate according to any one of claims 1 to 4, **characterised in that** the cover layer contains polymers, preferably polyolefins, copolymers of polyolefins, or polystyrene, inorganic fillers, inorganic anti-blocking agents, lubricants, or mixtures of these substances as additives.

6. Elastic laminate according to claim 4 or 5, **characterised in that** the proportion of the non-elastomer additives contained in the cover layer is less than 50 wt. %, preferably less than 30 wt. %, with reference to the mass of the cover layer, in each case.

7. Elastic laminate according to any one of claims 1 to 6, **characterised in that** the elastomer support layer has a thickness between 20 µm and 200 µm, and that the thickness of the elastomer cover layer is less than 20 µm.

8. Elastic laminate according to any one of claims 1 to 7, **characterised in that** the textile outer layer is glued to the cover layer of the elastic film over its entire area.

9. Elastic laminate according to any one of claims 1 to 8, **characterised in that** the textile outer layer is glued to the cover layer at point-shaped or line-shaped contact surfaces, or connected by means of thermobonding.

10. Elastic laminate according to any one of claims 1 to 9, **characterised in that** the stretchable region of the outer layer is dimensioned in such a manner that the laminate can be stretched by at least 100% with a stretching force of less than 4 N/cm.

11. Elastic laminate according to any one of claims 1 to 10, **characterised in that** the elastic film is structured in three layers, and a textile outer layer of nonwoven is laminated onto both sides of the elastic film, in each case.

## Revendications

1. Stratifié élastique pour bandes de fermeture ou petites attaches élastiques sur des couches, à base
d'un film élastique coextrudé, qui présente une couche porteuse élastomère et au moins une couche de recouvrement, et
au moins une couche extérieure textile en non-tissé,
la couche de recouvrement présentant une épaisseur de couche faible par rapport à la couche porteuse, la couche extérieure étant appliquée par contrecollage sur la couche de recouvrement et la couche de recouvrement étant à base d'un matériau élastomère, lequel présente de la même façon que la couche porteuse élastomère, contrairement aux thermoplastiques, après une contrainte de traction à la température ambiante, une fraction élastique marquée et contient des additifs pour la modification des propriétés de surface, **caractérisé en ce que** le matériau élastomère pour la couche de recouvrement et la couche porteuse est à base d'un élastomère thermoplastique choisi dans le groupe des copolymères en blocs, des polyuréthanes élastomères ou à base de mélanges de ces élastomères, **en ce que** le matériau en non-tissé de la couche extérieure est à base de fibres non élastomères, qui présentent une déformation permanente significative après un étirage, et **en ce que** la couche extérieure présente une zone extensible qui a été générée par étirage transversal du stratifié dans une fente de cylindres en forme de méandre entre deux cylindres à profiler qui s'engagent l'un dans l'autre

2. Stratifié élastique selon la revendication 1, **caractérisé en ce que** le matériau élastomère de la couche de recouvrement correspond au matériau élastomère de la couche porteuse.

3. Stratifié élastique selon une revendication 1 ou 2, **caractérisé en ce que** la couche porteuse contient des additifs pour améliorer l'aptitude au collage entre la couche porteuse et la couche de recouvrement.

4. Stratifié élastique selon l'une des revendications 1 à 3, **caractérisé en ce que** la couche de recouvrement contient des anti-bloquants et/ou des agents de glissement comme additifs pour la modification des propriétés de surface.

5. Stratifié élastique selon l'une des revendications 1 à 4, **caractérisé en ce que** la couche de recouvrement contient comme additifs des polymères, de préférence des polyoléfines, des copolymères, des polyoléfines ou du polystyrène, des agents de charge inorganiques, des anti-bloquants inorganiques, des agents de glissement ou des mélanges de ces produits.

6. Stratifié élastique selon la revendication 4 ou 5, **caractérisé en ce que** la fraction des additifs non élastomères inclus dans la couche de recouvrement est inférieure à 50 % en poids, de préférence inférieure à 30 % en poids, à chaque fois par rapport à la masse de la couche de recouvrement.

7. Stratifié élastique selon l'une des revendications 1 à 6, **caractérisé en ce que** la couche porteuse élastomère présente une épaisseur comprise entre 20 µm et 200 µm et **en ce que** l'épaisseur de la couche de recouvrement élastomère est inférieure à 20 µm.

8. Stratifié élastique selon l'une des revendications 1 à 7, **caractérisé en ce que** la couche extérieure textile est collée à plat avec la couche de recouvrement du film élastique.

9. Stratifié élastique selon l'une des revendications 1 à 8, **caractérisé en ce que** la couche extérieure textile est collée sur des surfaces de contact ponctuelles ou linéaires avec la couche extérieure textile ou est reliée par liage thermique.

10. Stratifié élastique selon l'une des revendications 1 à 9, **caractérisé en ce que** la zone extensible de la couche extérieure est dimensionnée de telle sorte que le stratifié est extensible d'au moins 100 % avec une force d'extension de moins de 4 N/cm.

11. Stratifié élastique selon l'une des revendications 1 à 10, **caractérisé en ce que** le film élastique est conçu avec trois couches et à chaque fois une couche extérieure textile en non-tissé est appliquée par contrecollage sur les deux côtés du film élastique.
